# EUROPEAN PATENT SPECIFICATION

(11) **EP 1 529 213 B1**
(45) Date of publication and mention of the grant of the patent: **20.12.2006**
(21) Application number: 03784694.6
(22) Date of filing: 07.08.2003
(51) Int. Cl.: G01N 33/487, G01N 33/50, C12Q 1/06, G01N 1/40, B01D 61/00

(54) **THE METHOD AND APPARATUS FOR DETERMINING THE NUMBER OF LIVING CELLS IN A TEST FLUID**
VERFAHREN UND VORRICHTUNG ZUR BESTIMMUNG DER ANZAHL VON LEBENDEN ZELLEN IN EINER TESTFLÜSSIGKEIT
PROCEDE ET DISPOSITIF PERMETTANT DE DETERMINER LE NOMBRE DE CELLULES VIVANTES DANS UN FLUIDE D'ESSAI

(30) Priority: 12.08.2002 NL 1021258
(43) Date of publication of application: 11.05.2005
(73) Proprietor: Nederlandse Organisatie voor Toegepast-Natuurwetenschappelijk Onderzoek TNO, 2628 VK Delft (NL)
(72) Inventor: VAN DUIJN, Albert, NL-2341 BV Oegstgeest (NL); DRAAIJER, Arie, NL-3704 BM Zeist (NL)
(74) Representative: Winckels, Johannes Hubertus F.
(86) International application number: PCT/NL2003/000569
(87) International publication number: WO 2004/015413

(56) References cited:
- EP-A- 0 612 850
- WO-A-00/57178
- WO-A-01/69243
- US-A- 5 137 031
- US-A- 5 976 892
- PATENT ABSTRACTS OF JAPAN vol. 006, no. 019 (C-090), 3 February 1982 (1982-02-03) & JP 56 140898 A (KYOWA HAKKO KOGYO CO LTD), 4 November 1981 (1981-11-04)

## Description

The invention relates to a method for determining the number of living cells in a test fluid, to an apparatus for such a method and to the use thereof.

An important quantity in the research field of cell biology and microbiology is cell density. This quantity, expressed in cells/volume unit, greatly determines the effects of cell populations perceptible to humans. An algal bloom, for instance, is defined as the visible manifestation of free floating algae or discernible coloring of surface water and/or a cell density of algae higher than 2,000 cells/ml of water. These high densities *inter alia* cause problems for surface water at the moment the bloom ends since microbial degradation makes the water virtually anoxic.

The importance of cell density does not only play a role in water quality monitoring but also in the cut flower trade. The bacterial contamination of stems and vascular systems and the density of bacterial cell populations in the vase water greatly affect the duration of the vase life of cut flowers.

Further, a rapid determination of cell densities is generally desired as a part of product quality control, hygiene monitoring, wastewater purification, horticultural practice, toxicological (soil) research and HACCP process monitoring and food safety.

As a measure for the cell density of a cell suspension, one can choose, for instance, the light extinction or light transmission through a cell suspension. Such an approach results in a method in which extinction or transmission of light of a specific wavelength (e.g. 550-600 nm) is used to determine the turbidity of a cell suspension.

However, at low cell densities and/or with very small cells and/or presence of other particles, the interaction with the light beam is not sufficient. Therefore, bacterial cell suspensions having densities of less than 10⁷ cells/ml cannot be tested in this manner.

In the past decades, many methodologies have been developed to determine the density of microorganisms. In this context, it is important to realize that there is a difference in urgency between determining densities of cells as with the above-described method for determining the turbidity of a sample, and densities of living cells, which cannot be determined by means of turbidity measurements.

Foods which contain bacteria are, in most cases, only health-threatening if the bacteria are alive. However, the latter is by no means always the case. If a food with high bacterial cell density has been pasteurized, still, many bacterial cells can be found in it by means of microscopic methods, but these hardly constitute a hazard to health. Although it is undesired from the viewpoint of hygiene and quality control that large numbers of dead cells are present in a food, in most cases, only the number of living cells is of interest to the researcher.

Bacteria, or more in general, microorganisms, are generally found at relatively low population densities of 10³ - 10⁷ cells/ml in the ambient environment, but can already be health-threatening even at these and even lower densities.

A very suitable method for determining the number of living cells at low cell density makes use of a culture method. Examples of this are the MPN (most probable number) method and plate count method. Both have the disadvantage that the incubation period needed for growth makes the method very slow. Therefore, a determination of the number of living cells at low density by means of culture techniques takes a very long time and improvements are much desired.

If it were possible for determinations of the number of living cells at low cell density to be done in a rapid manner, a possible contamination could already be determined at an early stage and measures could already be taken at an early stage.

Another method for determining the cell density of living cells makes use of epifluorescence microscopic techniques or a flow cytometric method in which individual cells can be counted and can also be specifically stained to determine the viability and/or the identity. Disadvantages of these methods are that the determinations require a very high level of expertise, that the equipment required for these is very costly, and that the methods are very sensitive to interfering, in particular particulate, substances in the test fluid, so that only bacterial populations with an initial density of more than 10⁵ cells/ml can be analyzed.

From the state of the art, methods are also known in which the presence of living cells can be determined by making use of a specific bioluminescence reaction between luciferin/luciferase and ATP. Here, the presence of ATP exclusively found in living cells is determined by means of a luminometer. The amount of light released during the reaction reflects the level of the microbial load present. By using a microscopic system in combination with the capture of the microorganisms on a filter, it is possible to carry out accurate counts of living cells. However, a disadvantage of this ATP bioluminescence method is that it is costly due to the reagents required. In addition, the intracellular ATP is not available for the luciferase and needs to be released from the cells before it can be measured. This is achieved by means of lysis of the cells by means of a suitable detergent. Therefore, this method is destructive.

A general problem of the methods for determining the number of living cells in a test fluid is that they are time-consuming and/or expensive.

WO 00/57178 discloses a system for detecting living cells comprising a step of concentrating the cells. Measurement is however performed in a separate array of wells.

It is an object of the present invention to provide a rapid and inexpensive method for determining the number of living cells in a test fluid.

Thus, the present invention provides a method for determining the number of living cells in a test fluid, in which, prior to measuring a metabolic rate of these cells by means of at least one measurement, the cells are concentrated in a reduced volume of the test fluid.

The present invention also provides an apparatus for determining the number of living cells in a test fluid by means of a method according to the invention.

Figs. 1 and 2 show embodiments of an apparatus for determining the number of living cells in a test fluid according to the invention.

Fig. 3 shows an example of how a signal of a measurement in an apparatus according to the invention can be obtained.

Fig. 4 shows a graph resulting from a measurement of oxygen consumption on a test fluid before and after concentration of a bacterial cell suspension.

In a first aspect, the present invention relates to a method for determining the number of living cells in a test fluid. According to the invention, such a method comprises the step of concentrating cells in a reduced volume of the test fluid. Contrary to the methods from the state of the art, this step has the advantage that the cells are not necessarily separated from their original growth environment. A result of this is that not just the potential activity of the cells is measured, but that also the actual activity of the cells in their environment can be determined.

Concentrating the cells in a reduced volume of the test fluid according to the invention is done using a filtration device, hereinafter to be referred to as a concentrator. The concentrator comprises a fluid container and a plunger, which plunger is provided, at its end, with a filter impermeable to the cells. The step of concentrating according to a method of the invention is carried out by placing a test fluid in the fluid container and pressing the filter through the test fluid by means of the plunger. The positive pressure forces a part of the test fluid through the filter, with the cells being left behind in the reduced volume of the test fluid.

Preferably, the step of concentrating the cells in a reduced volume of the test fluid is carried out to a concentration of 2 to 100,000 times the original density, more preferably to a concentration of 100 to 10,000 times the original density.

A method according to the invention further comprises the step of measuring a metabolic rate of the cells by means of at least one measurement in the reduced volume of the test fluid.

For determining a metabolic rate of the cells, a measurement can be carried out for a large number of parameters. For instance, very suitably, the formation of acid can be determined by measuring the pH. Also, the conversion rate or absorption rate of a natural or synthetic food substrate can be determined. A person skilled in the art is capable of choosing suitable (food) substrates which can be used to determine the metabolic rate of the cells. For this purpose, *inter alia* fluorescent analogs of enzyme substrates are commercially available. Preferably, the respiration rate is determined by measuring the changing oxygen content in the reduced volume of the test fluid.

Since, in many cases, it is possible to determine the initial value of the parameter to be measured in a manner other than by means of measurement, a metabolic rate can, in principle, already be determined by carrying out only one measurement.

Such a measurement can comprise an electrical measurement, as a conductivity measurement, an electrochemical measurement, as by means of a pH measurement, an optical measurement, such as a fluorescent or chemiluminescent measurement or an optochemical measurement. Preferably, an optochemical measurement is used in the present invention.

It is generally known that, for determining the metabolic rate of the cells by means of optical measurement, specific dyes can be used. For instance, the dehydrogenase activity of the cells can be determined by means of a tetrazolium salt, such as 5-cyano-2,3-ditolyl tetrazolium chloride (CTC), 2-(p-iodophenyl)-3-(p-nitrophenyl)-5-phenyl tetrazolium chloride (INT), 3-(4,5-dimethylthiazol-2-yl)-2,5-diphenyl tetrazolium bromide (MTT), 3-(4, 5-dimethylthiazol-2-yl)-5-(3-carboxymethoxyphenyl)-2-(4-sulfophenyl)-2H-tetrazolium (MTS) or 2,3-bis-(2-methoxy-4-nitro-5-sulfophenyl)-2H-tetrazolium-5-carboxanilide (XTT). It is also possible to use, for instance, an intracellular esterase assay with fluorescein diacetate (FDA) to determine the metabolic rate of the cells. Also, discoloration of methylene blue or trypan blue, a fluorescent fatty acid probe such as 11-dansylamino undecanoic acid (DAUDA) or a redox indicator such as resazurin can be used. A comprehensive description of suitable dyes can be found *inter alia* in the product catalog of Molecular Probes, Inc., Eugene, OR, US. In general, prior to the measurement, suitable dyes are added to the test fluid in an amount prescribed by the manufacturer. There is a great deal of literature available on the use and the manner of using dyes for determining metabolic rates and activities.

Preferably, extracellular pH indicators or oxygen indicators in combination with an optical measurement according to the invention are used to determine the rate of acid formation or the respiration rate as a measure for the metabolic rate of cells.

Great preference is given to a method for determining a metabolic rate of cells in which an optochemical or chemo-optical sensor is used to determine a change in the chemical composition of the test fluid as a result of the metabolic activity of the cells. Here, the optical sensor needs to be in contact with the reduced volume of the test fluid.

An optochemical sensor can be used to measure, for instance, the oxygen content, the glucose concentration or the pH or another suitable chemical parameter of the test fluid.

Optochemical sensors are known to a person skilled in the art. For instance, optochemical sensors as described in US 5,541,113, US 5,611,998, US 5,866,433, EP 1 199 556, or US 6,254,829 can be used. Preferably, an optochemical sensor as described in WO 01/69243 is used and a measurement according to the invention comprises the measurement of the oxygen content or a change thereof in a reduced volume of the test fluid.

A method according to the invention further comprises the step of calculating the number of living cells in the test fluid. For calculating the number of living cells in the test fluid with a first volume, the present invention provides a method step in which a value for the metabolic rate of the cells in the reduced volume is determined. This value can be determined by comparing the measured metabolic rate in the reduced volume with a corresponding value in a priorly made calibration curve. In this calibration curve, the value of the metabolic rate of the cells is plotted as a function of their density in a reference fluid.

An example of such a calibration curve comprises values for the respiration rate or for another metabolic rate of cells such as they are found or such as they are expected to be found in the sample to be tested. The calibration curve needs to comprise metabolic rates such as they will be determined in the test situation. To carry out this method step according to the invention, a parameter of the test fluid which will be measured needs to be determined or chosen in advance.

In a method according to the invention, the calibration curve shows the reference values for the metabolic rate to be measured as a function of the density of the cells. Therefore, a calibration curve can be drawn up very suitably by measuring metabolic rates at different cell densities. The calibration curve will comprise at least the metabolic rate measured at two cell densities. Preferably, the calibration curve also comprises values for the metabolic rate in the vicinity of the cell density to be expected in the reduced volume of the test fluid.

When drawing up a calibration curve, use can be made of the same test fluid as that in which the test measurement takes place, but another suitable fluid in which a metabolic rate can be measured can also serve as a reference fluid for drawing up a calibration curve. Preferably, the test fluid itself is used when producing a calibration curve.

In principle, a method according to the invention can be carried out with all cell types. Both of human cells and animal and plant cells, as well as of cell organelles (such as for instance mitochondria), but also of protozoa, fungi, yeasts, archaea or bacteria, the number of living cells in a test fluid can be determined. The present invention is eminently suitable for use in microbiological fields. Therefore, in a method according to the invention, preferably, the numbers of living bacteria are determined.

In a second aspect, the invention relates to an apparatus for determining the number of living cells in a test fluid by means of an above-described method.

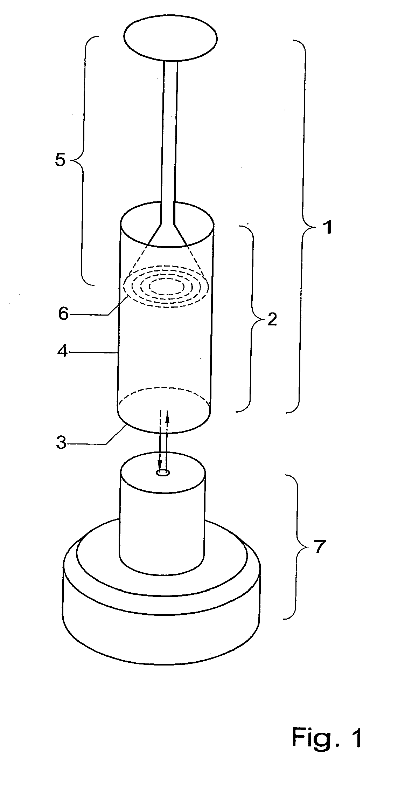

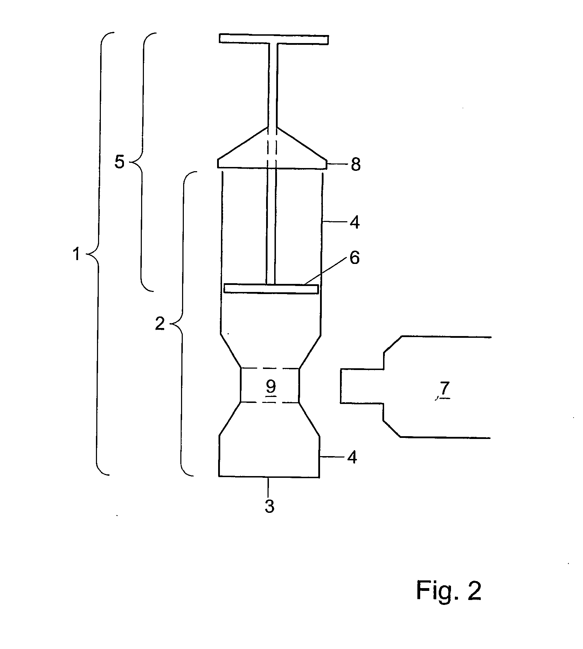

Possible embodiments of such an apparatus are shown in Figs. 1 and 2. The apparatus comprises a concentrator (1) and a measuring device (7). The concentrator (1) itself comprises a fluid container (2) for receiving a test fluid with cells. The container (2) is provided with a bottom (3) and adjoining walls (4). A suitable volume of the fluid container (2) can vary between approximately 10 µl and approximately 1 liter. Preferably, the volume of the fluid container (2) is between approximately 1 ml and approximately 250 ml, more preferably between 10 ml and 60 ml.

In case of use of an optochemical sensor which is in contact with the reduced volume, an optochemical sensor can be very suitably arranged on the bottom (3) of the fluid container (2). In that case, the measuring device (7) will be positioned in relation to the bottom (3) of the fluid container (2) as visible in Fig. 1.

However, an optochemical sensor can also be arranged at other positions in a fluid container (2) according to the invention, as long as there is contact between the sensor and the reduced volume of the test fluid. In that case, the measuring device (7) will be correspondingly positioned in relation to the optochemical sensor.

In all cases in which a measurement on a reduced volume of the test fluid is carried out by use of optical methods, fluid container (2) needs to be at least partly optically transparent to enable an optical measurement by means of measuring device (7).

In case of use of a dye for measuring a metabolic rate of cells in a reduced volume of the test fluid, in most cases, the dye will be homogeneously distributed over the reduced volume of the test fluid. For carrying out an optical measurement, at least a part of fluid container (2) can have the character of a cuvette (9) with optical properties, for instance in that, in at least a part of the walls (4) and/or the bottom (3), a quartz glass is used (see Fig. 2). For this purpose, in principle, any light-transmitting material can be used. Also, in some cases, it can be useful to arrange the fluid container (2), at the position where measurement on the reduced volume of the test fluid is done, such that an optical path of optionally known length is provided.

The concentrator (1) further comprises a plunger (5) which can be introduced into the fluid container (2) and which is movable in a fluid-closed manner along the walls (4) of the fluid container (2) in the direction of the bottom (3) of the fluid container (2). The plunger (5) is provided with a filter (6) impermeable to the cells. The filter (6) needs to be transmissive to the test fluid. This makes it possible to concentrate the cells in a decreasing volume of the test fluid. Depending on the size of the cells whose metabolic rate will be measured, the pore size of the filter can be adjusted.

In a method according to the invention, it is important that the plunger (5) is not moved all the way to the bottom (3) of the fluid container (2). By keeping the plunger (5) with filter (6) at some distance from bottom (3), a reduced volume of the fluid is realized. The degree to which the test fluid can be concentrated can be very suitably defined by stopping the movement of the plunger (5) at a predefined fixed distance from the bottom (3). For this purpose, for instance, projections (8) can be provided in the walls (4) of the fluid container (2) or to the plunger (5) itself (see Fig. 2). Preferably, the movement of the plunger (5) along the walls (4) of the fluid container (2) is limited to such a distance from the bottom (3) that a reduced volume of between 1 µl and 10 ml, preferably between 10 µl and 1 ml, more preferably between 50 µl and 150 ml is left.

In addition to the fact that, in a method according to the invention, an apparatus as described hereinabove can be used in which the fluid flow relative to the filter is substantially perpendicular, an alternative embodiment can comprise, for instance, the use of tangential flow filtration for the concentration of cells in a reduced volume of the test fluid. In this manner, a very large amount of fluid of 10 - 100 liters can be concentrated within a very short time and be further processed according to a method of the invention.

Filters which can be used in a method or apparatus according to the invention are capable of separating cells from fluid. Suitable filters are, for instance, membrane filters of polycarbonate, polyethylene terephthalate (PET), cellulose acetate, nitrocellulose or Teflon, optionally in combination with a coarse pre-filter, such as a glass fiber filter, or in combination with a support filter, such as a polycarbonate filter with an underlying nitrocellulose membrane.

Depending on the size of the cells to be concentrated, the pore size can be chosen by a person skilled in the art such that a separation between cells and fluid takes place.

A filter suitable for measurement of bacteria will have a pore size of between 0.05 and 5 µm. Preferably, for measurement of bacteria, a filter with a pore size of 0.1 to 1 µm, more preferably between 0.25 and 0.4 µm, is used.

According to an alternative embodiment of an apparatus according to the invention, it is possible to use filters with different pore sizes at a fixed distance from each other, which can result in different compartments with different cell sizes therein. By measurement of the metabolic rate of the cells in different compartments, cell numbers of different organisms can be determined.

In case of optical measurement, the measuring device (7) comprises optical conversion instruments for converting light from the test fluid to a measuring signal corresponding to a predetermined parameter of the test fluid which determines the metabolic rate of the cells.

The measuring device (7) preferably measures the consumption of oxygen as a metabolic parameter using a fluorescent oxygen-sensitive coating as an optochemical sensor. This coating can consist of, for instance, a gas-permeable polymer with a fluorescent dye embedded therein. The fluorescence of this dye can, for instance, be very suitably inversely proportional to the oxygen pressure in the fluid to be measured. The fluorescent dye can, for instance, act according to the principle of dynamic quenching. Without wishing to be bound to any theory, this principle works such that the fluorescent molecules in the excited state return to the ground state after collision with the oxygen molecule without fluorescent radiation being released. The more oxygen molecules, the higher the chances of collision and the lower the measured fluorescence.

In a method and apparatus according to the present invention, it is possible to determine the fluorescence intensity of the fluorescent dye in the fluorescent oxygen-sensitive coating or optochemical sensor. However, more preferably, the fluorescence lifetime is determined. In the case of use of, for instance, dynamic quenching in combination with a fluorescent oxygen-sensitive coating, the fluorescence lifetime is also inversely proportional to the oxygen concentration. Measuring the fluorescence lifetime has the advantage that this embodiment is less susceptible to interference.

In a measuring arrangement for determining, for instance, oxygen metabolism, the oxygen-sensitive coating which can be used for this purpose could be very suitably arranged in the part of the concentrator where the bacteria are concentrated, that is, in that part of the fluid container (2) which is in contact with the reduced volume of the test fluid.

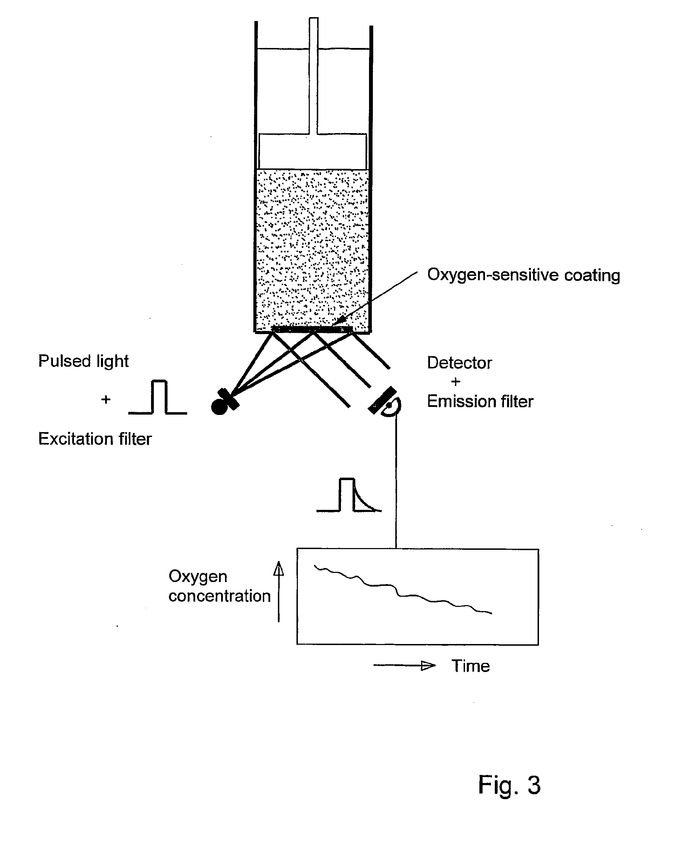

For carrying out a measurement, the concentrator (1), or that part on which the measurement can take place very suitably, can be contacted with a measuring device (7). Preferably, this measuring device (7) is capable of measuring the change in oxygen concentration in the reduced volume of the test fluid. Preferably, a measuring device (7) comprises a light source for this purpose, for instance a LED, and a detector; for instance a Si photodiode. Such a measuring device is described *inter alia* in WO 01/69243. The light from the light source can be administered continuously, but can also be very suitably administered in a pulsed manner to, for instance, an optochemical sensor present in the concentrator (1). A person skilled in the art is supposed to be familiar with the different optical techniques to carry out fluorescence measurements on an optochemical sensor as used in a method and apparatus according to the invention. For instance, pulsed light from the light source can irradiate an oxygen-sensitive coating via a so-called excitation filter to measure fluorescence lifetime. The fluorescence light emitted by the fluorescent oxygen-sensitive coating can be intercepted by the detector via an emission filter. By means of electronics intended for this purpose and a central data processing unit, this intercepted light can be very suitably used to determine a change in, for instance, the oxygen concentration, and from this, for instance, a bacterial cell density. An example of how a signal can be obtained from such a measurement is shown in Fig. 3.

A method and apparatus according to the invention can be used for determining the number of living cells in a solid substance, such as a meat product, a flower stem, a soil sample, a sediment, a biofilter or reactor bed or a flour product.

A method and apparatus according to the invention can also be used for determining the number of living cells in a semi-solid substance, such as a pudding, a tooth paste, a butter, but also in a fluid such as an oil or a watery fluid.

In the case of a solid substance, a semi-solid substance or an oil, it will be desirable to prepare a test fluid which can be used in the present invention from these samples to be tested. A person skilled in the art is familiar with methods to prepare a test fluid for use in the present invention from the different samples. In many cases, the cells will be extracted from a solid or semi-solid phase with a suitable amount of test fluid. A very usable method makes use of a stomacher for this purpose, but other manners of extraction, such as shaking, blending, mashing or mixing with water or with a test fluid, optionally in combination with a filtration step in which the cells to be tested can pass through the filter and end up in the filtrate to separate them from larger polluting particles such as sand, can also be used to prepare a test fluid containing the cells to be tested.

In most cases, it will be possible to use the test substance as a test fluid in the present invention without further processing. Especially water samples such as seawater, surface water or groundwater samples, flower water samples or water from a wastewater purification plant or a growth medium can be used directly without substantial processing. If required, a coarse filtration can be carried out to reduce the suspended fraction to the freely suspended cells as much as possible. However, this is not necessary and therefore it is possible to also determine the numbers of cells adhering to detritus or to other material by means of a method according to the invention.

If a sample to be tested is a solid or semi-solid test substance or oil, a test fluid according to the invention preferably comprises a watery fluid which is an extract of such a sample. If a sample to be tested is a watery test substance, the test substance can be used directly as a test fluid in a method according to the invention.

The test substance, or the sample to be tested as it is processed to a test fluid which can be used in a method or apparatus according to the invention, can contain between 1 and 10¹¹ cells per ml or gram of the test substance. A test fluid containing between 10² and 10⁵, preferably between 10³ and 10⁴ cells per ml of test fluid is very suitable for use in a method and apparatus according to the invention. A person skilled in the art is considered to be familiar with the numbers of cells to be expected as they can be found in the samples tested in his technical field. Therefore, a person skilled in the art will be capable of choosing a suitable volume of test substance to prepare a test fluid for use in a method and apparatus according to the invention on the basis of this.

At any moment before or during the method, compounds or substances can be added to a first or reduced volume of the test fluid. For instance, prior to the concentration step, dyes can be added to the test fluid. Also, certain growth substrates or growth inhibitors can be added to test their effect on the metabolic rate of the cells. This can especially be important if it is desired to determine the numbers of a specific subpopulation of cells in a test fluid by means of a method according to the invention. For instance, numbers of anaerobic cells can be determined by using substances which inhibit the metabolism of aerobic cells. A person skilled in the art will be capable of carrying out suitable variations of the method according to the invention, which variations are considered to fall within the scope of the present invention.

Embodiments of methods and the apparatus of the present invention can find application in *inter alia* product quality control, hygiene monitoring, water quality monitoring, wastewater purification, horticultural practice, cut flower trade, toxicological assays (for instance in soil research), scientific research, process monitoring as a part of e.g. a HACCP program in multifarious industries, including the pharmaceutical, cosmetics and toiletry industries, the food, dairy and beverage industries, and in water and environmental research.

The invention will now be illustrated in and by the following examples, which should not be construed as being limitative.

### Example 1.

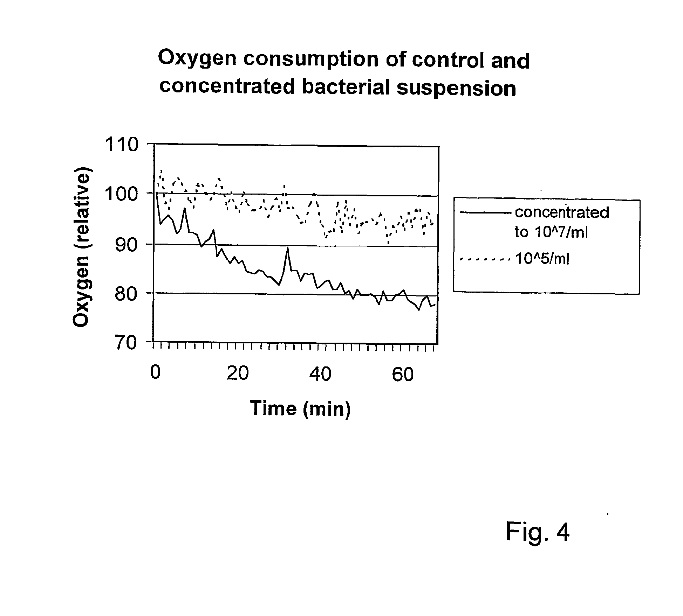

A suspension of 10⁵ *Escherichia coli* bacteria per ml was placed in the fluid container in an apparatus according to Fig. 1 (e.g. a Uniprep filter from Whatman company). On the bottom of the fluid container, a oxygen-sensitive fluorescent dye was provided. Without pressing the plunger downwards, the oxygen content in the suspension was measured in time. For this purpose, a fluorescence lifetime measurement was used. The dye and the manner of measurement were as described in WO 01/69243. The course of the oxygen concentration decrease due to oxygen consumption of the bacteria is shown in Fig. 4 (dotted line).

After this, this experiment was repeated with a same suspension (10⁵ bacteria/ml), but now the cell suspension was concentrated to 10⁷ per ml by pressing downwards the plunger provided with a filter impermeable to said cells. After this, the course of the oxygen concentration in the test fluid was again measured. The continuous line in Fig. 4 shows that, in this case, the oxygen level decreases much faster than in the unconcentrated suspension. This illustrates that the principle of concentrating the suspension in combination with the measurement of a metabolic parameter can be used to determine the relation between metabolic activity and initial cell density more rapidly and more accurately.

## Claims

1. A method for determining the number of living cells in a test fluid by measuring a metabolic rate of said cells by means of at least one measurement, wherein the cells are concentrated in a reduced volume of the test fluid, **characterized in that** the concentration of the cells and the measurement of the metabolic rate are performed in the same fluid container, and the volume is reduced by movement of a plunger, comprising a filter which is impermeable for said cells.

2. A method according to claim 1, wherein the number of living cells in the test fluid is determined by comparison of the measured metabolic rate with a priorly made calibration curve, in which the value of said metabolic rate of said cells is plotted as a function of their density.

3. A method according to claim 1 or 2, wherein said cells are human cells, animal cells, plant cells, cell organelles, protozoa, fungi, yeasts, archaea or bacteria.

4. A method according to claim 1 or 2, wherein said cells are bacteria.

5. A method according to any one of the preceding claims, wherein said at least one measurement comprises an optical measurement.

6. A method according to any one of the preceding claims, wherein said metabolic rate comprises the respiration rate, or the conversion rate or absorption rate of a natural or synthetic substrate or a dye.

7. A method according to claim 5 or 6, wherein the optical measurement comprises a fluorescent or chemiluminescent measurement.

8. A method according to any one of claims 5-7, wherein an optical sensor is used which is in contact with said reduced volume of said test fluid.

9. A method according to claim 8, wherein said optical sensor comprises a chemo-optical substance.

10. A method according to any one of the preceding claims, wherein oxygen is measured.

11. An apparatus for determining the number of living cells in a test fluid by means of a method according to any one of claims 1-10, comprising a concentrator (1) and a measuring device (7), which concentrator (1) comprises a fluid container (2) for receiving a test fluid with cells, which fluid container (2) is provided with a bottom (3) and adjoining walls (4), which concentrator (1) further comprises a plunger (5) which can be introduced into the fluid container (2) and which is movable in a fluid-closing manner along the walls (4) of the fluid container (2) in the direction of the bottom (3) of the fluid container (2) and wherein the plunger (5) is provided with a filter (6) impermeable to said cells, which measuring device (7) comprises optical conversion elements for converting light from the test fluid to a measuring signal which corresponds to a predetermined parameter of the test fluid.

12. Use of an apparatus or method according to any one of the preceding claims in product quality control, hygiene monitoring, water quality monitoring, wastewater purification, horticultural practice, cut flower trade, toxicological assays for instance in soil research or in process monitoring.

## Patentansprüche

1. Verfahren zum Bestimmen der Anzahl lebender Zellen in einer Testfluid, indem eine Metabolismusrate der Zellen mittels wenigstens einer Messung gemessen wird, wobei die Zellen in einem reduzierten Volumen des Testfluids konzentriert werden, **dadurch gekennzeichnet, dass** die Konzentration der Zellen und die Messung der Metabolismusrate in demselben Fluidbehälter durchgeführt werden und dass das Volumen durch Bewegen eines Kolbens reduziert wird, der einen für die Zellen undurchlässigen Filter aufweist.

2. Verfahren nach Anspruch 1, wobei die Anzahl lebender Zellen in dem Testfluid durch Vergleich der gemessenen Metabolismusrate mit einer zuvor erstellten Kalibrationskurve bestimmt wird, in der der Wert der Metabolismusrate der Zellen als Funktion ihrer Dichte aufgetragen ist.

3. Verfahren nach Anspruch 1 oder 2, wobei die Zellen menschliche Zellen, Tierzellen, Pflanzenzellen, Zellorganellen, Protozoen, Pilze, Hefen, Archäen oder Bakterien sind.

4. Verfahren nach Anspruch 1 oder 2, wobei die Zellen Bakterien sind.

5. Verfahren nach einem der vorhergehenden Ansprüche, wobei die wenigstens eine Messung einer optischen Messung umfasst.

6. Verfahren nach einem der vorhergehenden Ansprüche, wobei die Metabolismusrate die Respirationsrate, oder die Konversionsrate oder die Absorptionsrate eines natürlichen oder synthetischen Substrats oder eines Farbstoffes, umfasst.

7. Verfahren nach Anspruch 5 oder 6, wobei die optische Messung eine Fluoreszenz- oder Chemolumineszenz-Messung umfasst.

8. Verfahren nach einem der Ansprüche 5 - 7, wobei ein optischer Sensor verwendet wird, der in Kontakt mit dem reduzierten Volumen des Testfluids ist.

9. Verfahren nach Anspruch 8, wobei der optische Sensor eine chemo-optische Substanz aufweist.

10. Verfahren nach einem der vorhergehenden Ansprüche, wobei Sauerstoff gemessen wird.

11. Vorrichtung zum Bestimmen der Anzahl lebender Zellen in einem Testfluid mittels eines Verfahrens nach einem der Ansprüche 1 - 10, mit einem Konzentrator (1) und einem Messgerät (7), wobei der Konzentrator (1) einen Fluidbehälter (2) zur Aufnahme eines Testfluids mit Zellen hat, wobei der Fluidbehälter (2) mit einem Boden (3) und anschließenden Wänden (4) versehen ist und wobei der Konzentrator (1) weiter einen Kolben (5) aufweist, der in den Fluidbehälter (2) eingeführt werden kann und der in fluiddichter Weise entlang der Wände (4) des Fluidbehälters (2) in die Richtung auf den Boden (3) des Fluidbehälters (2) zu beweglich ist, und wobei der Kolben (5) mit einem Filter (6) versehen ist, der für die Zellen undurchlässig ist, wobei das Messgerät (7) optische Umwandlungselemente zum Umwandeln von Licht aus dem Testfluid in ein Messsignal aufweist, das einem vorgegebenen Parameter des Testfluids entspricht.

12. Verwendung einer Vorrichtung oder eines Verfahrens nach einem der vorhergehenden Ansprüche bei der Produktqualitätskontrolle, Hygieneüberwachung, Wasserqualitätsüberwachung, im Gartenbau, Schnittblumenhandel, toxikologischen Untersuchungen (zum Beispiel bei der Bodenuntersuchung) oder bei der Prozessüberwachung.

## Revendications

1. Procédé pour déterminer le nombre de cellules vivantes dans un fluide test en mesurant un taux métabolique desdites cellules par l'intermédiaire d'au moins une mesure, dans lequel les cellules sont concentrées dans un volume réduit du fluide test, **caractérisé en ce que** la concentration des cellules et la mesure du taux métabolique sont effectuées dans le même conteneur de fluide, et le volume est réduit par déplacement d'un plongeur, comportant un filtre qui est imperméable vis-à-vis desdites cellules.

2. Procédé selon la revendication 1, dans lequel le nombre de cellules vivantes dans le fluide test est déterminé en comparant le taux métabolique mesuré à une courbe d'étalonnage réalisée antérieurement, dans lequel la valeur dudit taux métabolique desdites cellules est tracée en fonction de leur densité.

3. Procédé selon la revendication 1 ou 2, dans lequel lesdites cellules sont des cellules humaines, cellules animales, cellules végétales, organelles cellulaires, protozoaires, champignons, levures, archéobactéries ou bactéries.

4. Procédé selon la revendication 1 ou 2, dans lequel lesdites cellules sont des bactéries.

5. Procédé selon l'une quelconque des revendications précédentes, dans lequel ladite au moins une mesure comporte une mesure optique.

6. Procédé selon l'une quelconque des revendications précédentes, dans lequel ledit taux métabolique comporte le rythme respiratoire, ou le taux de conversion ou le taux d'absorption d'un substrat naturel ou synthétique ou d'un colorant.

7. Procédé selon la revendication 5 ou 6, dans lequel la mesure optique comporte une mesure de fluorescence ou de chimioluminescence.

8. Procédé selon l'une quelconque des revendications 5 à 7, dans lequel un capteur optique est utilisé qui est en contact avec ledit volume réduit dudit fluide test.

9. Procédé selon la revendication 8, dans lequel ledit capteur optique comporte une substance chimio-optique.

10. Procédé selon l'une quelconque des revendications précédentes, dans lequel de l'oxygène est mesuré.

11. Appareil pour déterminer le nombre de cellules vivantes dans un fluide test par l'intermédiaire d'un procédé selon l'une quelconque des revendications 1 à 10, comportant un dispositif de concentration (1) et un dispositif de mesure (7), lequel dispositif de concentration (1) comporte un conteneur de fluide (2) pour recevoir un fluide test avec des cellules, lequel conteneur de fluide (2) est muni d'un fond (3) et de parois attenantes (4), lequel dispositif de concentration (1) comporte de plus un plongeur (5) qui peut être introduit dans le conteneur de fluide (2) et qui est mobile, d'une manière renfermant le fluide, le long des parois (4) du conteneur de fluide (2) dans la direction du fond (3) du conteneur de fluide (2) et dans lequel le plongeur (5) est muni d'un filtre (6) imperméable pour lesdites cellules, lequel dispositif de mesure (7) comporte des éléments de conversion optique pour convertir une lumière provenant du fluide test en un signal de mesure qui correspond à un paramètre prédéterminé du fluide test.

12. Utilisation d'un appareil ou d'un procédé selon l'une quelconque des revendications précédentes pour le contrôle de qualité de produit, la surveillance d'hygiène, la surveillance de la qualité d'eau, la purification d'eaux usées, la pratique horticole, le commerce de fleurs coupées, des essais toxicologiques, par exemple pour la recherche podologique, ou pour la surveillance d'un traitement.
